# EUROPEAN PATENT APPLICATION

(11) **EP 2 609 813 A1**
(43) Date of publication of application: **03.07.2013**
(21) Application number: 11196138.9
(22) Date of filing: 30.12.2011
(51) Int. Cl.: A23L 1/30, A61K 35/74

(54) **Lactobacillus reuteri DSM 17938 for the development of the enteric nervous system**

(71) Applicant: Nestec S.A., 1800 Vevey (CH)
(72) Inventor: Bergonzelli Degonda, Gabriela, 1030 Bussigny (CH); Faure, Magali, 1072 Forel (CH); Kusy, Nicole, 1674 Montet (CH)
(74) Representative: Corticchiato, Olivier

(57) **Abstract**

The invention relates to *Lactobacillus reuteri* DSM 17938, and compositions comprising *Lactobacillus reuteri* DSM 17938, for promoting the healthy development and/or repair of the enteric nervous system in mammals. Humans or animals and, in particular, a foetus, pre- term or term-born infant, toddler or child may benefit from the invention. The invention may be especially beneficial to those infants having experienced IUGR, or having a low, or very low birth weight, and/or suffered from enteric nervous system growth retardation either in utero or, during or after birth. The administration of to *Lactobacillus reuteri* DSM 17938 promotes normal and healthy neuronal and glial development. It also ensures healthy neuronal differentiation in the peripheral nervous system.

## Description

### Field of the Invention

The present invention relates generally to the field of neuronal health, neuronal protection and neuronal development. The invention specifically relates to administration of probiotic bacteria capable of promoting the enteric neuronal development in infants, especially preterm and low birth weight infants.

### Background to the Invention

The nervous system is a highly complex network composed of neuronal and glial cells. It is present in all mammalian species and is composed of the central nervous system (brain and spinal cord) and the peripheral nervous system (somatic, autonomous and enteric nervous system).

The central nervous system drives the cognitive functions (memory, attention, perception, action, etc). Together with the peripheral nervous system, it has a fundamental role in the control of behaviour. The somatic nervous system is responsible for coordinating the body's movements (under conscious control). The autonomous nervous system maintains homeostasis in the body activities without conscious control (heart rate, etc). Finally, and as part of the latter system, the enteric nervous system directly controls the gastrointestinal tract functions. These include the gastrointestinal barrier and immune function, motility, absorption, digestion and exocrine / endocrine secretions, which contribute to the protection of the intestine from any type of injury and to digestive comfort [Neunlist, M. et al. (2008); Neuro-glial crosstalk in inflammatory bowel disease, J Intern. Med, 263: 577-583], [Burns, A.J. et al. (2009); Development of the enteric nervous system and its role in intestinal motility during fetal and early postnatal stages, Semin. Pediatr. Surg.,18:196-205], [Tapper, E.J. (1983); Local modulation of intestinal ion transport by enteric neurons, Am J Physiol., 244:G457-68].

The nervous system develops during gestation and then refines to a mature, functional network during the post natal period.

Immaturity or delayed maturation of the nervous system leads to delayed establishment and adequate functioning of the important biological functions that it regulates. In particular, it leads to intestinal dysfunctions after birth, including a reduced digestive/absorptive capacity, gastrointestinal reflux, a slower intestinal transit/harder stools that might lead to constipation, an impaired gut colonization, a weak intestinal barrier function that increases infection and allergy risks, all this leading to food intolerance (therefore need of parenteral nutritional support) and necrotizing enterocolitis in most severe cases, and gastrointestinal discomfort.

This can be observed in infants such as:
- Preterm infants, low birth weight (<2500 g) and very low birth weight infants (<1500 g); New J. Gastrointestinal development and meeting the nutritional needs of premature infants Am J Clin Nutr 2007;85(2): 629S-634S
- Premature or term-born infants having experienced an intrauterine growth retardation (IUGR) that occured following any adverse events during the gestation (smoking of the mother, medication of the mother, low placenta quality, abnormal placenta positioning, malnutrition of the mother and the foetus, excessive stress/anxiety of the mother, etc); - [Shanklin D.R. and Cooke R.J. (1993); Effects of intrauterine growth on intestinal length in the human foetus, Biol Neonate, 64:76-81], [Neu, J. (2007); Gastrointestinal development and meeting the nutritional needs of premature infants, Am. J. Clin. Nutr., 85(2): 629S-634S], [Brandãoa, M.C.S. et al., (2003); Effects of pre- and postnatal protein energy deprivation on the myenteric plexus of the small intestine: a morphometric study in weanling rats, Nutr. Res., 23: 215-223].
- Any neonate and young infant showing nervous system growth retardation following for example hypoxemia-ischemia at birth or any other adverse event [Taylor, C.T. and Colgan S.P. (2007); Hypoxia and gastrointestinal disease, J. Mol. Med. (Berl.), 85:1295-300], [Barrett R.D. et al. (2007); Destruction and reconstruction: hypoxia and the developing brain, Birth Defects Res C Embryo Today, 81:163-76].
- Any neonate and infant showing gastrointestinal dysfunctions (digestive disorders, motility disorders, gastrointestinal reflux, slow gastrointestinal transit, oral feeding intolerance), Hirschsprung's disease, and inflammation affecting the gastrointestinal tract (such as Necrotisis enterocolitis) and obstruction pathologies [Burns A.J. et al. (2009); Development of the enteric nervous system and its role in intestinal motility during fetal and early postnatal stages, Semin. Pediatr Surg., 18(4):196-205].

It is known that, in humans, the neural crest cells, from which the enteric nervous system derives, develop *in utero* in the foetus very early on after conception (from 7.5 weeks of development) [Burns, A. J. and Thapar, N. (2006); Advances in ontogeny of the enteric nervous system, Neurogastroenterol. Motil., 18, 876-887] The enteric nervous system has been shown to undergo major changes after birth up until the age of 6 years old, with more minor changes continuing to occur up until the age of ten years old [Wester, T. et al. (1999); Notable postnatal alterations in the myenteric plexus of normal human bowel, Gut, 44:666-674]. Thus, if the foetus, neonate or infant has experienced nervous system growth retardation, it is desirable that this retardation be reversed quickly so that the nervous system development "catches up" to a normal level. It is desirable that any damage caused to the enteric nervous system be repaired as quickly as possible, so that the growing foetus or infant experiences as little as possible any gastrointestinal dysfunctions or other pathologies associated with an immature or damaged enteric nervous system. Thus, the healthy development of the enteric nervous system in the foetus, neonate and growing child helps to control correct establishment and maintenance of the gut barrier function, thus prevent inflammatory pathological states associated with intestinal dysfunction as well as decreasing the risk of infection and allergy (Neulist et *al*. 2008)

Thus, peri-, and/or postnatal interventions correspond to a promising approach to ensure the healthy development of the enteric nervous system. Interventions during pregnancy/lactation may have considerable advantages in terms of convenience and compliance compared to child-directed interventions.

There is a need to promote and support the healthy development and/or repair of the enteric nervous system at the earliest possible stage during gestation as well as during the early phases of their newborn life, when the nervous system is rapidly maturing. Because the nervous system continues to develop during the early years of childhood (up until the age of approximately ten years old), this need for continued support exists throughout this period.

The scientific literature reports on the use of probiotics to impact positively on the health of neonates, and infants, in particular, with respect to reducing inflammation, protecting against infection, and positively impacting on bowel habits and gastrointestinal motility. For a recent general review see [Dobrogosz, W. J., Peacock, T.J. and Hassan, H. M. (2010); Evolution of the probiotic Concept: From Conception to Validation and acceptance in Medical Science, Advances in Applied microbiology, 72: 1-41].

Human derived *Lactobacillus reuteri* is considered as endogenous organism of the human gastrointestinal tract and is present on the mucosa of the gastric corpus and antrum, duodenum and ileum [Reuter, G. (2001); The Lactobacillus and Bifidobacterium microflora of the human intestine: composition and succession, Curr. Issues Intest. Microbiol, 2: 43-53] and [Valeur, N. et al. (2004); Colonization and immunomodulation by Lactobacillus reuteri ATCC 55730 in the human gastrointestinal tract, Appl. Environ. Microbiol., 70: 1176-1181]. There are reports of *L. reuteri* as a promising therapy for many different conditions including diarrheal disease [Saavedra, J. (2000); Am. J. Gastroenterol, 95: S16-S18], infantile colic [Savino, F. et al. (2007); Lactobacillus reuteri (American Type Culture Collection Strain 55730) versus simethicone in the treatment of infantile colic: a prospective randomized study, Pediatrics, 119: e124-e130], eczema [Abrahamsson, T.R. et al. (2007); Probiotics in prevention of IgE-associated eczema: a double-blind, randomized, placebo-controlled trial, J Allergy Clin. Immunol., 119: 1174-1180] and H. pylori infection [Imase, K. et al. (2007); Lactobacillus reuteri tablets suppress Helicobacter pylori infection - a double-blind randomised placebo-controlled crossover clinical study, Kansenshogaku Zasshi, 81: 387-393]. A recent study on four *L. reuteri* strains, DSM 17938, ATCC PTA4659, ATCC PTA 5289 and ATCC PTA 6475 demonstrated that the strains differentially modulated lippopolysaccharide induced inflammation in small intestinal epithelial calls and in the ileum of newborn rats.

In a double blind randomized study carried out on thirty preterm newborns, reported in 2008 [Indrio, F. et al. (2008); The effects of probiotics on feeding tolerance, bowel habits, and gastrointestinal motility in preterm newborns, J. Pediatr., 152(6):801-6], the newborns receiving *Lactobacillus reuteri* ATCC 55730 showed a significant decrease in regurgitation and mean daily crying time and a larger number of stools compared with those given placebo.

To date, there has been no report on the effect of probiotics on the neuronal development in neonates.

Thus, in the light of these cited prior art documents, there is a need for a further progress in area of probiotic administration as a way to improve the health of newborn babies, young infants, toddlers and children.

There is, in particular, a need to support the healthy development of the enteric nervous system in the neonates, infants and young children, in order to best prepare them for gastro intestinal challenges such as changes of diet, chemical (for instance medication) or physical (abrasion) injuries, infections, inflammatory/immune reactions, etc., as well as to enhance the future maturation of their enteric nervous system during later life.

The present invention applies to all mammals, including animals and humans.

### Summary of the invention

The invention relates to *Lactobacillus reuteri* DSM 17938 for promoting the healthy development and/or repair of the enteric nervous system in neonates. Humans or animals and, in particular, a foetus, pre- term or term born infant, toddler or child may benefit from the invention. The invention may be especially beneficial to those infants having experienced IUGR, or having a low, or very low birth weight, and/or suffered from enteric nervous system growth retardation either *in utero* or, during or after birth.

The administration of to *Lactobacillus reuteri* DSM 17938 promotes normal and healthy neuronal and glial development. It also ensures healthy neuronal differentiation in the peripheral nervous system.

*Lactobacillus reuteri* DSM 17938 is administered directly to the infant or toddler in its pure form, or diluted in water or breast milk, in a food supplement, or in a milk fortifier, or any milk support used during trophic feeding, in an infant formula, or in a milk based drink. The *Lactobacillus reuteri* DSM 17938 is administered to the infant, toddler or child as a daily dose of from 1x10³ to 1x10¹², preferably, 1x10⁷ to 1x10¹¹ cfu (cfu = colony forming unit).

The invention relates to a composition comprising *Lactobacillus reuteri* DSM 17938 for promoting the healthy development and/or repair of the enteric nervous system in neonates.

### Brief Description of the Drawings

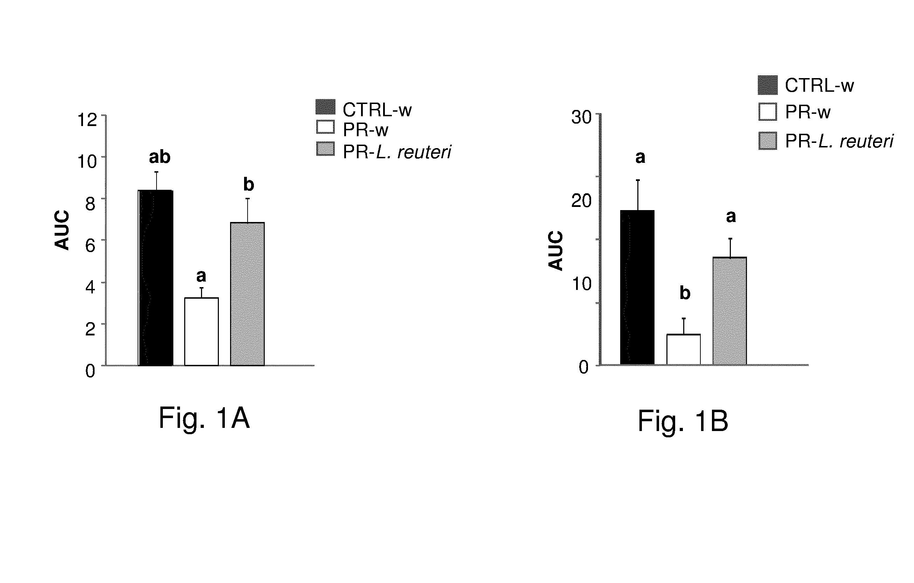

### Figure 1 Electrical field stimulation of the ex-vivo contractile response

### A: Jejunum

Tensions (area under the curve (AUC)) obtained by isometric contractions of jejunum in response to electrical field stimulation at 10 Hz in CTRL-w, PR-w and PR-*L.reuteri* (DSM 17938) pups at sacrifice (post-natal day 14). The results are medians ± SEMedian, n=5 (CTRL-w and PR-w) or 6 (PR-*L.reuteri*). Medians without a common letter differ, *P*<0.05.

### Figure 1 Electrical field stimulation of the ex-vivo contractile response

### B: Colon

Tensions (area under the curve (AUC)) obtained by isometric contractions of colon in response to electrical field stimulations at 5 Hz in CTRL-w, PR-w and PR*-L.reuteri* (DSM 17938) pups at sacrifice (post-natal day 14). The results are medians ± SEMedian, n=5 (CTRL-w and PR-w) or 6 (PR*-L.reuteri*). Medians without a common letter differ, *P*<0.05.

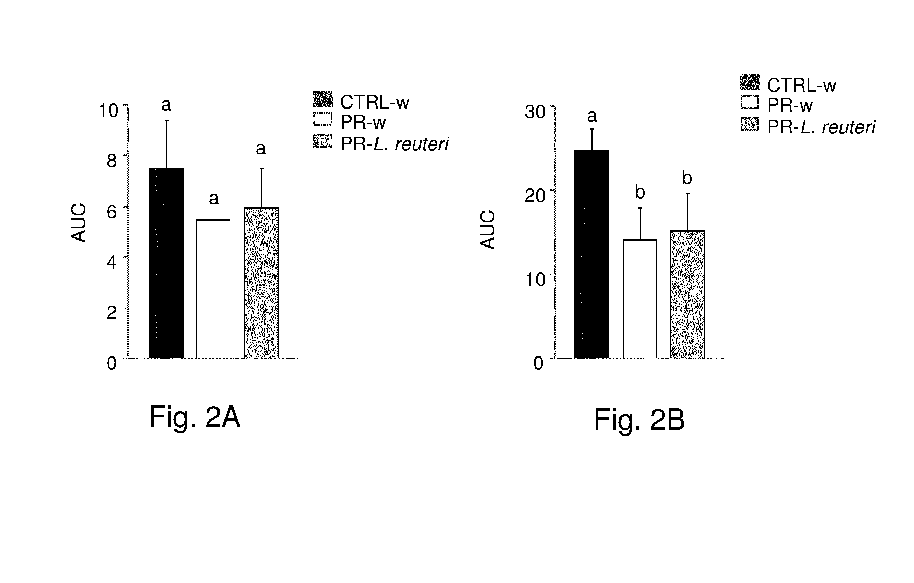

### Figure 2 Acetylcholine stimulation of the ex-vivo contractile response

### A: Jejunum

Tensions (area under the curve (AUC)) obtained by isometric contractions of jejunum in response to acetylcholine at concentration of 10⁻⁵ M in CTRL-w, PR-w and PR*-L.reuteri* (DSM 17938) pups at sacrifice (post-natal day 14). The results are medians ± SEMedian, n=5 (PR-w) or 6 (CTRL-w and PR*-L.reuteri*). Medians without a common letter differ, *P*<0.05.

### Figure 2 Acetylcholine stimulation of the ex-vivo contractile response

### B: Colon

Tensions (area under the curve (AUC)) obtained by isometric contractions of colon in response to acetylcholine at concentration of 10⁻⁵ M in CTRL-w, PR-w and PR-*L.reuteri* (DSM 17938) pups at sacrifice (post-natal day 14). The results are medians ± SEMedian, n=5 (PR-w) or 7 (PR-*L.reuteri*) and 9 (CTRL-w). Medians without a common letter differ, *P*<0.05.

### Detailed description

### Definitions:

In this specification, the following terms have the following meanings:
*"Probiotic"* means microbial cell preparations or components of microbial cells with a beneficial effect on the health or well-being of the host. [Salminen, S. et *al*. (1999); Probiotics: how should they be defined, *Trends Food Sci. Technol*., 10 107-10]. The definition of probiotic is generally admitted and in line with the WHO definition. The probiotic can comprise a unique strain of micro-organism, a mix of various strains and/or a mix of various bacterial species and genera. In case of mixtures, the singular term "probiotic" can still be used to designate the probiotic mixture or
preparation. For the purpose of the present invention, micro-organisms of the genus *Lactobacillus* are considered as probiotics.

*"Prebiotic"* generally means a non digestible food ingredient that beneficially affects the host by selectively stimulating the growth and/or activity of micro-organisms present in the gut of the host, and thus attempts to improve host health.

*"Pre-term infant"* means an infant born before 37 weeks gestation.

*"Term Born Infant"* means an infant born after 37 weeks gestation.

*"Toddler"* means a child from when he can walk up to three years old.

*"Young child"* means a child from the age of three up to ten years old. *Lactobaccilus reuteri* DSM 17938, referred to as *L. reuteri* throughout the text, is the *L. reuteri* strain owned by Biogaia AB, Sweden, having the scientific strain designation DSM 17938, formerly *L. reuteri* ATCC 55730. The DSM identification refers to the DSMZ Deutsche Sammlung von Mikroorganismen und Zellkulturen GmbH Inhoffenstr. 7b, D - 38124 Braunschweig, Germany. DSM 17938. Deutsche Sammlung von Mikroorganismen und Zellkulturen GmbH Inhoffenstr. 7b D - 38124 Braunschweig - Germany.

The present invention provides a probiotic, *Lactobacillus reuteri* DSM 17938, for use by administration for the promotion of the healthy development of the mammalian enteric nervous system.

The administration of the probiotic may be to a foetus via the mother. It may also be to a pre-term or term-born infant either directly or via mothers' milk. The administration may be also be to a young child, generally up to the age of ten years old, or the equivalent age in an animal.

The administration of the *L. reuteri* to the young mammal, which may be human (foetus, infant, toddler or young child) or animal has a positive effect on the development of their enteric nervous system, allowing the system to mature and develop normally. This effect is especially beneficial for those who have experienced, for example, intra uterine growth retardation (IUGR) that may have occurred following any adverse event during the gestation (for example, active or passive smoking of the mother, medication of the mother, low placenta quality, abnormal placenta positioning, malnutrition of the mother and/or the foetus, etc).

The present inventors have found that *L. reuteri* and/or *L. reuteri* containing compositions of the present invention may be used to promote neuronal cell development, functionality, survival, plasticity and differentiation and to protect neuronal cells against degeneration, as shown by promotion of the expression of proteins associated with these biological activities. Such degeneration may follow, for example, any stress situations, such as those affecting the foetus (*in utero*) such as IUGR, mentioned above, or the newborns (hypoxia-ischemia at birth, oxygen therapy and hyperoxia, inflammation, need for parenteral support, etc), or any cause leading to oxidative stress. *L. reuteri* was found to promote neuronal survival and/or limit or prevent neuronal death of enteric neuronal cells, and to promote neuronal growth which is important, for example, in developmental processes.

In infants, the *L*. *reuteri* and/or the *L*. *reuteri* containing compositions of the present inventions may be used to protect the enteric nervous system from any stress, e.g., occurring during the neuronal development period, and - consequently - to limit and/or prevent stress-induced neuronal growth retardation and associated intestinal dysfunctions.

Thus, *L. reuteri* may be administered, in the context of the present invention, when there has been already observed a retardation in the development of the enteric nervous system or, prophylactically, when no such retardation has as yet been observed.

By administering the *L. reuteri* according to the present invention, the intestinal microbiota of the young mammal is affected propitiously, and the enteric nervous system of the treated subject develops healthily. The risk of the infant, or toddler, or young child thus treated suffering from pathologies associated intestinal dysfunction after birth is reduced. These pathologies include a reduced digestive/absorptive capacity, gastrointestinal reflux, a slower intestinal transit/harder stools and constipation, an impaired gut colonization, and a weak intestinal barrier function. These latter states increase risk of infection and allergy, thus leading to food intolerance (therefore need of parenteral nutritional support). Other pathologies associated with intestinal dysfunction after birth are necrotizing enterocolitis, and gastrointestinal discomfort. The beneficial effect of *L. reuteri* on the healthy development of the mammalian enteric nervous system is elaborated upon in the paragraphs below.

### Doses of probiotic:

The probiotic may be administered as a daily dose and in the form of a composition. The daily doses of *L*. *reuteri* administered to the expectant or breast feeding mother is from 1x10⁶ to 1x10¹² cfu, preferably 1x10⁸ to 1x10¹¹ cfu (cfu = colony forming unit). The daily dose suitable for newborn babies ranges from 1x10³ to 1x10¹², preferably, 1x10⁷ to 1x10¹¹ cfu.

Thus, *L. reuteri* may be present in the composition in a wide range of percentages provided that it delivers the beneficial effect described. However, preferably, the *L. reuteri* is present in the composition in an amount equivalent to between 1 x10³ and 1x10¹² cfu/g of dry composition. Preferably, for administration to the expectant or lactating mother, the probiotic is present in an amount equivalent to between 1x10⁴ to 1x10¹¹ cfu/g of dry composition. The amount of probiotic present per gram of dry composition for administration to the neonates, toddlers and children may be lower, preferably, 1x10⁶ to 1x10⁹, and, of course, the daily doses described above should be respected.

The above doses include the possibilities that the bacteria are live, inactivated or dead, or even present as fragments such as DNA or cell wall materials. In other words, the quantity of bacteria which the formula contains is expressed in terms of the colony forming ability of that quantity of bacteria as if all the bacteria were live irrespective of whether they are, in fact, live, inactivated or dead, fragmented or a mixture of any or all of these states.

### Method of administration:

(i) Administration to expectant mothers:
   The composition can be administered to the expectant mothers by various ways as long as it induces a contact between the composition and the gastro-intestinal tract of the females. Preferably, the composition is orally administered as part of the food, drinks or dietary supplements of the expectant mothers. The composition can also be administered in a pharmaceutical composition. Preferably the administration is oral. However, in pathological conditions or when enteral feeding is otherwise used, the administration of the composition can be added to the enteral feeding.
(ii) Administration to newborn progeny:
   The *L*. *reuteri* can also be administered orally directly to the progeny alone (pure or diluted in water or mother's milk for example) as a supplement (for example as a human milk fortifier supplement), or as a pharmaceutical or neutriceutical composition, or as an ingredient in an infant milk formula. Such a formula may be an infant "preterm formula" if the progeny is born before term or has a low birth weight, a "starter formula" or a "follow-on formula". The formula may also be an hypoallergenic (HA) formulas in which the cow milk proteins are hydrolysed. An example of such starter formula is given in **Example 2.**
(iii) Administration to toddlers and young children:
   The *L*. *reuteri* can also be administered orally to toddlers and young children in the form of a pharmaceutical or neutriceutical composition, growing-up milk, milk based drinks, food supplements, milk based yoghurts, desserts and puddings, biscuits and cereal bars, cereals and fruit based drinks.
(iv) Administration to animals:
   The *L*. *reuteri* may also be administered orally to animals alone, or in water or in the form of a food supplement, a pharmaceutical or neutriceutical composition, or milk or pet food.

### Administration with other compounds:

The *L*. *reuteri* can be administered alone (pure, or diluted in water or milk, including breast milk for example) or in a mixture with other compounds (such as dietary supplements, nutritional supplements, medicines, carriers, flavours, digestible or non-digestible ingredients). Vitamins and minerals are examples of typical dietary supplements. In a preferred embodiment, *L. reuteri* is administered in a composition, for example, an infant formula, together with other compounds that enhance the described beneficial effect on the young mammals. Such synergistic compounds may be carriers or a matrix that facilitates the *L*. *reuteri* delivery to the intestinal tract or they may otherwise enhance the effect of the composition on the enteric nervous system of the progeny. Such compounds can be other active compounds that synergistically or separately influence the development of the enteric nervous system in the infant and/or potentiates the effect of the probiotic. An example of such synergistic compounds is maltodextrin. One of the effect of maltodextrin is to provide a carrier for the probiotic, enhancing its effect, and to prevent aggregation.

Other examples of synergistic compounds that may be included in the compositions, especially infant formula, of the invention are prebiotic compounds. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon, where they are selectively fermented by the beneficial bacteria. Examples of prebiotics include certain oligosaccharides, such as fructooligosaccharides (FOS), cow milk oligosaccharides (CMOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®. Other examples of prebiotics that can be used in the context of the present invention include the group of oligosaccharides obtained from milk or other sources, optionally containing sialic acid, fructose, fucose, galactose or mannose. Preferred prebiotics are sialo-oligosaccharides (SOS), fructo-oligosaccharides (FOS), galacto-oligosaccharides (GOS), isomalto-oligosaccharides (IMO), xylo-oligosaccharides (XOS), arabino-xylo oligosaccharides (AXOS), mannan oligosaccharides (MOS), oligosaccharides of soy, glycosylsucrose (GS), lactosucrose (LS), sialyl-lactose (SL), fucosyl-lactose (FL), Lacto-N-Neotetraose (LNNT), lactulose (LA), palatinose-oligosaccharides (PAO), malto-oligosaccharides, gums and/or hydrolysates thereof, pectins, starches, and/or hydrolysates thereof. An infant formula according to the invention preferably further contains at least one prebiotic in an amount of 0.3 to 10% of the total weight of the dry composition.

The daily doses of carbohydrates, and all other compounds administered with the *L. reuteri* should always comply with the published safety guidelines and regulatory requirements. This is particularly important with respect to the administration to newborn babies, especially those born with low birth weight or very low birth weight.

The composition, for example infant formula, containing the *L*. *reuteri* may contain a protein source in an amount of not more than 4.0, 3.0 or 2.0 g/l00kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. In one embodiment, the protein content is between 30% and 80% whey proteins. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and betalactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The composition may also comprise a source of carbohydrates and/or a source of fat. The infant formula may contain a source of lipids. The lipid source may be any lipid or fat which is suitable for use in infant formulas. Preferred fat sources include palm olein, high oleic sunflower oil and high oleic safflower oil. The essential fatty acids, linoleic and α-linolenic acid may also be added as small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5: 1 to about 15: 1 ; for example about 8: 1 to about 10: 1.

An additional source of carbohydrate may be added to the nutritional composition. It preferably provides about 40% to about 80% of the energy of the nutritional composition. Any suitable carbohydrate may be used, for example sucrose, lactose, glucose, fructose, corn syrup solids, maltodextrin, or a mixture thereof.

Additional dietary fibre may also be added if desired. If added, it preferably comprises up to about 5% of the energy of the nutritional composition. The dietary fibre may be from any suitable origin, including for example soy, pea, oat, pectin, guar gum, acacia gum, fructooligosaccharide or a mixture thereof. Suitable vitamins and minerals may be included in the nutritional composition in an amount to meet the appropriate guidelines.

Examples of minerals, vitamins and other nutrients optionally present in the infant formula include vitamin A, vitamin B1, vitamin B2, vitamin B6, vitamin B 12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

The infant formula may optionally contain other substances which may have a beneficial effect such as fibres, lactoferrin, nucleotides, nucleosides, and the like.

One or more essential long chain fatty acids (LC-PUFAs) may be included in the composition. Examples of LC-PUFAs that may be added are docosahexaenoic acid (DHA) and arachidonic acid (AA). The LC-PUFAs may be added at concentrations so that they constitute greater than 0.01% of the fatty acids present in the composition.

One or more food grade emulsifiers may be included in the nutritional composition if desired; for example diacetyl tartaric acid esters of mono- and di- glycerides, lecithin and mono- or di-glycerides or a mixture thereof. Similarly suitable salts and/or stabilisers may be included. Flavours can be added to the composition.

### Administration period:

The duration of the administration may vary. While positive effects are expected with relatively short duration of administration (for example, daily administration during one to two weeks for newborns), longer durations are believed to provide an enhanced effect, or, at least, to maintain the effect in older infants (for example, a duration of three, five, eight or 12 months) or in young children (for example, a duration up to the age of 4 or 6 or even 10 years old). For administration to animals the corresponding durations apply.

The expectant mother may start to take the *L. reuteri* as soon as she is aware of her pregnancy. However, the administration period may also start before pregnancy starts, for example if the female is trying to become pregnant. Administration may start at any time after the pregnancy starts. It may start relatively late in the pregnancy, preferably at month 3, 4, 5, 6, 7 or 8 of the pregnancy, in the case of human pregnancy, or in corresponding periods for other mammals, or up to two weeks before the expected delivery date.

The period of administration can be continuous (for example, up to and including lactation up to weaning), or discontinuous. Continuous administration is preferred for a more sustained effect. However, it is speculated that a discontinuous pattern (for example, daily administration during one week per month, or during alternate weeks) can induce positive effects on the progeny.

The administration may cover at least part of the gestation period and at least part of the lactation period, or the equivalent period should the newborn not be breastfed. Preferably, the administration period to the expectant mother covers substantially the full length of the gestation period, although this may be less. Similarly, the administration period for the lactating mother preferably covers substantially the full length of the lactation period, although, again, this period may be less.

Preferably, the administration to the mother is by daily intake (to be taken once or twice a day), or weekly intake (to be taken one or twice a week).

The *L*. *reuteri* may be administered to the infant directly. This is the case particularly if the mother does not breastfeed or after she discontinues breastfeeding. However, an infant who is being breastfed may also receive the *L*. *reuteri* by direct administration.

Preferably, the administration to the infant is by daily intake. For example, if the *L. reuteri* is administered as an infant formula, the administration is with each feed, i.e. about four to about six times daily for infants less than year old, the number of feeds reducing with age. For infants older than one year, the administration may be less, once or twice a day. For toddlers and young children the administration may be daily or weekly (to be taken one or twice a week).

The administration to the infant, either via breastfeeding, or by direct administration, or both methods, may be continued up until the age of six months or even one year or longer. Thus, the *L. reuteri* may be administered during lactation if lactation takes place, or after partial or full weaning. Administration may continue to the infant through the toddler stage and even, up until the age of ten years old. It is known that the enteric nervous system continues to develop in children until this age [Wester, T. et al. (1999); Notable postnatal alterations in the myenteric plexus of normal human bowel, Gut, 44:666-674]. Thus, the present inventors speculate that the administration of *L*. *reuteri* may continue to have a positive effect up until this age.

### Effect of the L. reuteri administration:

*L. reuteri* administered to neonates promotes the healthy development of the enteric nervous system. In a rat model experiment detailed in **Example 1** the effect of *L*. *reuteri* administration on neuronal development was evaluated.

In this experiment, pups which had experienced maternal diet induced intra-uterine growth retardation (PR group) and pups which had not experienced IUGR (CTRL) were supplemented, from 2 days after birth, with water (controls; namely CTRL-w and PR-w) or *L. reuteri* (PR-*L.reuteri*).

2 weeks after birth, at sacrifice, the neuronal development was evaluated using two methods: (i) a targeted gene expression profiling approach in jejunum, and (ii) by measuring the ex-vivo contractile response of jejunum and colon to an electrical field stimulation (EFS). The latter method measures the contractile response induced by the enteric nervous system, and therefore the maturation and functional degree of the enteric nervous system.

The ex-vivo contractile response of jejunum and colon to acetylcholine was also evaluated, this measures the intestinal muscle contractile response itself, which therefore reflects the maturation and functional degree of the intestinal muscle. The latter experiment was performed as a control, to allow the inventors to separate the factors, i.e. nervous system and muscle, controlling the measured contractile responses.

### Ex-vivo contractile response to EFS of jejunum and colon:

In order to evaluate the neuronal maturation in the protein restriction model and following *L. reuteri* supplementation, the contractile response of jejunum and colon to EFS, which measures the implication/capacity of the neuronal network to stimulate the muscle contractility, was measured. The possible implication of any intestinal muscular maturation due to the protein restriction or following *L. reuteri* supplementation was evaluated by measuring the contractile response of jejunum and colon to acetylcholine.

The jejunum contractile response to EFS was reduced in PR-w as compared to CTRL-w at the frequency of 10 Hz (P=0.055) (**Figure 1A**). The jejunum contractile response to acetylcholine was slightly reduced, but not significantly (P=0.30), in PR-w as compared to CTRL-w (**Figure 2A**). Taken together, this reflects an impaired neuro-muscular interaction, and in particular an impaired neuronal maturation, in the jejunum of protein restricted pups.

The supplementation with *L. reuteri* increased significantly (P=0.017) the contractile response capacity of the jejunum at 10 Hz to a level statistically similar to controls (**Figure 1A**). This indicates a promoting effect of *L*. *reuteri* on the neuronal stimulatory capacity in the jejunum, which reflects a promotion of the neuronal development and function in the neonatal period. The maturation of the jejunum muscle compartment was indeed not significantly modified following the *L. reuteri* supplementation (P=1.00), since the jejunum contractile response capacity to acetylcholine remained unchanged as compared to PR-w **(****Figure 2A****).**

The colonic contractile response to EFS was significantly reduced in PR-w as compared to CTRL-w at 5 Hz **(****Figure 1B****).** The colonic contractile response to acetylcholine was significantly reduced (P=0.04) in PR-w as compared to CTRL-w **(****Figure 2B****).** Taken together, this reflects an impaired neuro-muscular interaction, with both an impairment of neuronal and muscle maturation, in the colon of protein restricted pups.

The supplementation with *L. reuteri* increased the colonic contractile response capacity at a frequency of 5 Hz (P=0.008) **(****Figure 1B****),** indicating again a stimulatory effect of *L*. *reuteri* on the neuronal development and function in the colon. The maturation of the colonic muscle compartment was indeed not significantly modified following the *L. reuteri* supplementation (P=0.43), as the colonic contractile response capacity to acetylcholine remained unchanged as compared to PR-w **(****Figure 2B****).**

Taken together, these data clearly show a beneficial effect of *L*. *reuteri* to promote neuronal development and function during the postnatal period. Based on these data, *L. reuteri* constitutes a new nutritional solution to promote and/or accelerate the intestinal neuronal development during the neonatal period.

### Expression of genes involved in the gut neuronal development:

In the rat model experiment of **Example 1,** the effect of protein restriction, as well as protein restriction followed by *L*. *reuteri* supplementation, on the expression of major genes involved in neuronal development biological pathways was studied in the jejunum, using a gene expression profiling approach. The genes studied are listed in **Table 2** and the data are presented in **Table 3.**

A significant difference in expression level between the treatment (protein restriction) and the supplementation with *L*. *reuteri* was observed for 11 of the genes expressed at a meaningful level, while 3 showed a tendency to be different which was not statistically significant. Genes that were significantly differentially expressed are those mainly involved in the maturation or differentiation of neurons and glial cells, or are markers of neuronal growth and plasticity.

The present inventors have previously shown (Cettour and Faure, manuscript in preparation) that compensatory mechanisms take place in protein restricted pups (PR) to help promote the cellular and neuronal development in the small intestine. The present data confirm these findings. Specifically, the gene expression of neurotrophic factors and their receptors, such as Gfra1 (GDNF family receptor alpha 1), Ntf4 (Neurotrophin 4), S100B (S100 calcium binding protein B) and GAP43 (growth associated protein 43), which is a marker for neuronal development, were significantly up-regulated in the PR-w group as compared to CTRL-w. This likely reflects a disequilibrium and a "stress" situation in the animals, which remains nevertheless insufficient to promote the neuronal maturation as indicated by our results following EFS stimulation.

The supplementation with *L. reuteri* significantly restored the expression level of some of these important genes to levels similar to CTRL-w (see **Table 3**). The genes affected are described below.

GAP43 is a neuronal growth and plasticity marker that is highly expressed in neuronal growth cones during development and is thus a marker for neuronal development [Benowitz, L.I. and Routtenberg, A. (1997); GAP-43: an intrinsic determinant of neuronal development and plasticity, Trends Neurosci., 20(2):84-91]. [Capone G.T. et al. (1991); Developmental expression of the gene encoding growth-associated protein 43 (Gap43) in the brains of normal and aneuploid mice, J. Neurosci. Res., 29(4):449-60].

Ntrk3, also known as TrkC, is a receptor mediating the multiple effects of several neurotrophic factor such as neuronal differentiation and survival, and which in particular binds the neurotrophin-3 that is of particular importance during the neonatal enteric nervous system development [Maisonpierre P.C. et al. (1990); Neurotrophin-3: a neurotrophic factor related to NGF and BDNF, Science, 23;247(4949 Pt 1):1446-51].

S100 is present in developing chicken neurons and Schwann cells and promotes motor neuron survival *in-vivo.* [Bhattacharyya, A., et al. (1992); S100B, a potent neurotrophic factor, is also a biomarker for glial cell development, J Neurobiol., 23(4):451-66].

The restoration of gene expression levels for these latter genes to that of the control animal levels indicates that supplementation with *L. reuteri* helps restore normal and healthy neuronal and glial development activity in the pups. Thus, the model demonstrates that administration of *L. reuteri* can help animals who have suffered IUGR recover to a "normal" or "healthy" state in terms of neuronal cell development, functionality, survival, plasticity and differentiation.

The gene expression level of SOX10 was significantly up-regulated following the supplementation with *L. reuteri.* SOX10 encodes a transcription factor of crucial importance for neuronal differentiation in the peripheral nervous system during the development. It is a marker for glial and neuronal progenitor cells highly present at embryonic stages and early postnatal development period [Wegner M. and Stolt, C.C. (2005); From stem cells to neurons and glia: a Soxist's view of neural development, Trends Neurosci., 25(11):583-8] and [Young, et al. (2003); Acquisition of neuronal and glial markers by neural crest-derived cells in the mouse intestine, The Journal of Comparative Neurology, 456:1-11]. Such stimulation of SOX10 expression by *L. reuteri* supplementation is thus likely to accelerate and/or promote the differentiation and functionality of neurons, thus favoring the rapid establishment of the neuronal network.

The modifications of gene expression observed in **Table 3** likely contribute to the observed promotion of the neuronal stimulatory responses to EFS in **Figure 1****.**

The *L. reuteri* strain is a probiotic isolated from the fecal microbiota of a breastfed child. Thus, with respect to a health promoting strategy trying to mimic as closely as possible the microbiota of breastfed children, the administration of *L. reuteri,* particularly to non-breast fed children, may provide an advantage over other strains that are not found in breastfed children.

### Example 1:

### Animal study (feeding and sacrifice) :

Animal experiments were conducted under authorization n°2120 granted by the Office Vétérinaire Cantonal, Etat de Vaud. Two months old female Sprague-Dawley rats were obtained after one week of gestation from Harlan, Barcelona. On the day of their arrival, rat dams were placed in individual cages and randomly assigned either to control (CTRL) or protein restricted (PR) groups. Animals had access to food and water *ad libitum* and were maintained in a 12 hr light/dark cycle.

Diets of CTRL and PR dams are detailed in Table 1. CTRL dams received a control diet containing 20 % of proteins (casein) fitting standard rat protein requirement during gestation (Reeves *et al.,* 1993). PR dams received a PR diet containing 10% of proteins (casein). Both diets were iso-caloric, the protein deficit being balanced by addition of corn starch.

### Diets

**Table 1: Composition of control (CTRL) and protein restricted (PR) AIN-93G diets**

| Components | CTRL | PR |
|---|---|---|
| Cornstarch | 53 | 63 |
| Caseine (K-Caseinate) | 20 | 10 |
| Sucrose | 10 | 10 |
| Soybean oil | 7 | 7 |
| Cellulose | 5 | 5 |
| Mineral mix AIN-93G | 4 | - |
| Mineral mix AIN-93M | - | 4 |
| Vitamin mix AIN-93 | 1 | 1 |
| Choline Bitartrate | 0.25 | 0.25 |
| L-Cysteine | 0.3 | 0.3 |
| Tert-buthylydroquinone | 0.0014 | 0.0014 |

CTRL and PR dams received their respective diets during both gestation and lactation until the day of sacrifice (postnatal day 14 (PND 14)).

On PND 2, pups were randomly assigned to dams from the same experimental group, and litter size was adjusted to 9 pups per dam with a minimal number of four to five males per litter.

From PND 2 till PND14, a daily hand/pipette feeding supplementation of water or *L. reuteri* containing solution was administered to control or treated groups, respectively. The volume of supplementations was gradually adapted to match the growth of rat pups (150 µl/ 100g body-weight).

The groups and diets were as follow:
1) CTRL-w: CTRL pups born from CTRL dams, receiving a supplementation of water,
2) PR-w: PR pups born from PR dams, receiving a supplementation of water,
*3) PR-L.reuteri:* PR pups born from PR dams, receiving a supplementation of *L. reuteri* DSM 17938 freeze-dried (1.10⁹ cfu/day).

At PND 14, a maximum of 10 pups from CTRL and PR groups were weighed and then sacrificed by decapitation after halothane anaesthesia.

### Ex-vivo contractile response of jejunum and colon to electrical field stimulation (EFS) :

Approximately 1 cm length segments of distal jejunum and distal colon were placed in cold Krebs' solution (CaCl₂ (5 mM), MgCl₂.6H₂ (1.2 mM), NaCl (120 mM), NaH₂PO₄.H₂O (1.2 mM), NaHCO₃ (15.5 mM), KCI (5.9 mM), phenol red (0.005 mM) and glucose (11.5 mM)) pre-oxigenated with 95% O₂/5% CO₂. The intestinal segments were suspended along their longitudinal axis in a tissue bath (50 ml volume) filled with Krebs solution oxigenated with 95% O₂/5% CO₂, at 37°C. One end of the muscle was attached to a stationary clamp. The other end was attached via an inelastic wire to an isometric force transducer. A stabilization period of 30 min was allowed to obtain spontaneous contractions. The tissue was then stretched to an initial length such that any further stretch would increase tension at rest. Isometric contractility in the distal jejunum and colon were induced by acetylcholine (Ach, 10⁻⁵ M) or by electrical field stimulation (EFS, 5-10 Hz). Ach was applied for 1 min. After completion of the dose-response curve, the strips were tested with 80 mM KCI to assure that they had maintained their ability to contract. The signals were digitally recorded on a computer using Powerlab Chart 3.4. The results were normalized by cross-sectional area after carefully blotting and weighing the tissue fragments. Non-linear fittings were performed using Prism 4.0 (GraphPad Software, Inc., San Diego, USA). To obtain the frequencies, an analysis of the power density spectra of the measured signal was carried out. A high pass filter, with a cut off frequency of 2 Hz (much smaller than the Nyquist frequency of 100 Hz) was applied. The Welch method was used for the computation of the power spectrum density with 1,3 seconds of overlapping, imposing a low frequency discretization: a serie of frequencies was defined between 0 and 2 Hz, equally spaced by 0.05 Hz. The calculations of the power spectrum density were made using the Goertzel algorithm. Three frequencies corresponding to the three largest power values below 2 Hz were chosen.

### Gene expression profiling :

Total RNA were extracted from jejunum of PR and CTRL pups using the phenol/chloroform method with the TriPure^{®} (Roche Diagnostics, Basel, Switzerland) reagent according to the manufacturer instructions. Briefly, frozen tissue samples (50-100mg) were homogenized in 1 ml of TriPure^{®} using the TissueLyser (Qiagen AG, Basel, Switzerland). Quantity and quality of the isolated RNA were measured using the RiboGreen RNA quantification kit (Invitrogen- Molecular Probes, Carlsbad, CA) and the RNA 6000 Nano LabChip kit (agilent Technologies) according to the instructions provided with the kits. The reverse transcription was performed using an oligo (dT15) primer (Promega, Madison, WI) and the ImProm-II(tm) reverse transcription system (Promega) according to the instructions provided by the manufacturer, with 1 µg total RNA.

**Table 2: List of genes analysed.**

| **Gene Symbol** | **Alias** | **Official Full Name** | **Function** |
|---|---|---|---|
| Bdnf | MGC105254 | Brain derived neurotrophic factor | Neurotrophic factor |
| Egf | - | Epidermal growth factor | Growth factor |
| Gap43 | Basp2/MGC15666 6 | Growth associated protein 43 | Neuronal maturation |
| Gdnf | - | Glial cell derived neurotrophic factor | Glial Maturation |
| Gfap | - | Glial fibrillary acidic protein | Glial Maturation |
| Gfra21 | - | GDNF family receptor alpha 1 | Glial Maturation |
| Gfra2 | Retl2 | GDNF family receptor alpha 2 | Glial Maturation |
| Gfra3 | - | GDNF family receptor alpha 3 | Glial Maturation |
| Gmfb | MGC93372 | Glia maturation factor, beta | Glial Maturation |
| Gmfg | - | Glia maturation factor, gamma | Glial Maturation |
| Igf1 | - | Insulin-like growth factor 1 | Growth & neurotrophic factor |
| Igf2 | IGFII/RNIGF2 | Insulin-like growth factor 2 | Growth & neurotrophic factor |
| Neurod 1 | - | Neurogenic differentiation 1 | Neurodifferenciation |
| Npffr2 | Gpr74/Npff2/Npg pr | Neuropeptide FF receptor 2 | Neuronal maturation factor |
| Ngf | Ngfb | Nerve growth factor (beta polypeptide) | Neurotrophic factor |
| Ngfr | LNGFR/RNNGFRR /p75/p75NTR | Nerve growth factor receptor (TNFR superfamily, member 16) | Neurotrophic receptor |
| Nrg1 | - | Neuregulin 1 | Neurodifferenciation |
| Nrg2 | NTAK_alpha2a/Nt ak | Neuregulin 2 | Neurodifferenciation |
| Ntf3 | - | Neurotrophin 3 | Neurotrophic factor |
| Ntf4 | NT4P/Ntf5 | Neurotrophin 4 | Neurotrophic factor |
| Ntrk1 | TrkA | Neurotrophic tyrosine kinase, receptor, type 1 | Neurotrophic receptor |
| Ntrk2 | RATTRKB1/TRKB1 /Tkrb | Neurotrophic tyrosine kinase, receptor, type 2 | Neurotrophic receptor |
| Ntrk3 | trkC | Neurotrophic tyrosine kinase, receptor, type 3 | Neurotrophic receptor |
| S100b | MGC93559/S100 P | S100 calcium binding protein B | Neurotrophic factor |
| Syp | Syp1 | Synaptophysin | Neurodevelopment |
| Sox10 | - | SRY (sex determining region Y)-box 10 | Neurodifferenciation |
| Tgfa | RATTGFAA/TGFA A | Transforming growth factor alpha | Growth factor |
| Tgfb1 | - | Transforming growth factor, beta 1 | Growth factor |
| Actb | Actx | Actin, beta | Controls |
| Rpl13a | - | Ribosomal protein L13A | Controls |
| Rplp1 | MGC72935 | Ribosomal protein, large, P1 | Controls |
| Gapdh | Gapd | Glyceraldehyde-3-phosphate dehydrogenase | Controls |
| RGDC | RGDC | Rat Genomic DNA Contamination | Controls |

The expression levels of major genes involved in the enteric nervous system development (see **Table 2)** were assessed by 2-step quantitative real-time RT-PCR using RT Profiler PCR Array System by Sybergreen (SABiosciences).

Measurements were done in duplicates using specific sets of primers and fluorescent TAMRA probes during the log-linear phase of a PCR reaction with the 7600HT TaqMan Fast Real-Time PCR System (Applied Biosystems) using Sequence Detection Software, version 2.2. The PCR reactions took place within 2-µl wells previously loaded with the specific primers and probes by the manufacturer into each of the 384 wells of the reaction card. The reaction was performed using a final cDNA sample concentration of 0.8 ng/µl with the TaqMan Universal PCR Master Mix (Applied Biosystems) containing the AmpliTaq Gold DNA Polymerase enzymes (Applied Biosystems), the nucleotides and the ROX fluorescent dye used as a passive load reference. The sequences of primers and probes used were designed and validated by Applied Biosystems and were taken from the Assay-on-Demand rat library. The relative expression level of each gene was normalized by the geometric average of control genes (those whose expression was statistically stable across experimental groups in our conditions).

**Table 3: Expression levels of genes in the jejunum of CTRL-w, PR-w and PR-L.reuteri pups at sacrifice (post natal day 14). The results are means ± SEM, n=6 (PR-w) or 8 (CTRL-w; PR-L.reuteri).**

| | **Expression levels** | | | | | | **Analysis of variance** | | | |
|---|---|---|---|---|---|---|---|---|---|---|
| | CTRL-w | | PR-w | | PR-*L reuteri* | | Probability levels | | | |
| | Means | SEM | Means | SEM | Means | SEM | Interaction | PR-w vs CTRL-w | PR-L reuteri vs PR-w | PR-*L reuteri vs* CTRL-w |
| Apoe | 989 | 246 | 1116 | 202 | 1109 | 263 | 0,437 | 0,495 | 0,204 | 0,512 |
| Gap43 | 145 | 22 | 272 | 27 | 163 | 30 | **0,022** | **0,009** | **0,023** | 0,641 |
| Gdnf | 7,3 | 1,2 | 5,8 | 1,1 | 5,2 | 2,6 | 0,990 | 0,891 | 0,946 | 0,940 |
| Gfra1 | 8,9 | 1,2 | 19,2 | 1,4 | 14,5 | 2,4 | **0,032** | **0,010** | 0,118 | 0,195 |
| Gfra2 | 37 | 4 | 56 | 10 | 53 | 11 | 0,512 | 0,270 | 0,695 | 0,435 |
| Gfra3 | 42 | 4 | 57 | 6 | 42 | 5 | 0,086 | 0,054 | 0,046 | 0,930 |
| Gmfb | 320 | 109 | 125 | 20 | 99 | 23 | **0,002** | **0,007** | 0,419 | **0,001** |
| Gmfg | 301 | 32 | 475 | 69 | 183 | 33 | **0,007** | 0,408 | **0,003** | **0,014** |
| Igf1 | 2,8 | 0,7 | 3,3 | 0,5 | 2,6 | 0,2 | 0,792 | 0,774 | 0,734 | 0,500 |
| Igf2 | 135 | 42 | 64 | 85 | 64 | 35 | 0,327 | 0,253 | 0,889 | 0,170 |
| Neurod1 | 10,8 | 1,2 | 9,1 | 0,9 | 10,1 | 1,8 | 0,897 | 0,773 | 0,646 | 0,853 |
| Ngf | 1,3 | 0,3 | 1,5 | 0,3 | 1,4 | 0,3 | 0,958 | 0,931 | 0,858 | 0,775 |
| Ngfr | 6,5 | 0,8 | 6,7 | 0,7 | 13,2 | 3,7 | 0,091 | 0,925 | 0,064 | 0,057 |
| Nrg1 | 33 | 9 | 18 | 2 | 34 | 10 | 0,107 | 0,040 | 0,122 | 0,537 |
| Nrg2 | 5,7 | 0,4 | 9,6 | 1,8 | 8,2 | 1,5 | 0,063 | 0,021 | 0,248 | 0,169 |
| Ntf3 | 1,4 | 0,2 | 1,3 | 0,2 | 0,6 | 0,1 | **0,000** | 0,268 | **0,003** | **0,000** |
| Ntf4 | 1,3 | 0,1 | 2,4 | 0,3 | 1,8 | 0,3 | **0,014** | **0,004** | 0,076 | 0,143 |
| Ntrk3 | 95 | 15 | 193 | 21 | 80 | 19 | **0,009** | **0,017** | **0,003** | 0,418 |
| S100b | 78 | 22 | 228 | 42 | 82 | 19 | **0,003** | **0,003** | **0,002** | 0,806 |
| Syp | 4,3 | 0,8 | 5,0 | 0,7 | 6,1 | 1,0 | 0,965 | 0,841 | 0,981 | 0,808 |
| Sox10 | 1,1 | 0,3 | 1,1 | 0,1 | 3,7 | 0,6 | **0,006** | 0,799 | **0,005** | **0,005** |
| Tgfa | 7,4 | 1,7 | 2,3 | 0,8 | 3,2 | 2,2 | 0,156 | 0,060 | 0,174 | 0,532 |
| Tgfb1 | 290 | 14 | 421 | 55 | 364 | 42 | **0,012** | **0,003** | 0,056 | 0,174 |
| Actb | 35739 | 2724 | 40604 | 3079 | 40339 | 2979 | 0,610 | 0,330 | 0,513 | 0,723 |
| Rpl13a | 8277 | 708 | 8238 | 632 | 6792 | 612 | 0,435 | 0,480 | 0,203 | 0,526 |
| Rplp1 | 29795 | 1613 | 40820 | 3012 | 28871 | 1915 | **0,002** | **0,005** | **0,001** | 0,361 |
| Gapdh | 1224 | 69 | 687 | 62 | 1062 | 337 | **0,012** | **0,017** | **0,004** | 0,520 |

### Statistics :

The effect of protein restriction was evaluated by comparing PR with CTRL groups. The effect of the *L. reuteri* supplementation was evaluated by comparing PR-*L. reuteri* with PR groups, and an eventual restoration to CTRL levels was evaluated by comparing PR-*L*. *reuteri* with CTRL groups.

Nonparametric methods were used to analyse the data (except for gene expression, see below). Wilcoxon rank sum test was used to test the differences between the treatments. Hodges-Lehmann estimate of the pair-wise treatment difference with its 95% confidence interval was also obtained. For gene expression, the statistical analyses were performed on the raw cycle threshold (Ct) counts, assumed to be log 2 values. A one-way Analysis of Variance (ANOVA) was applied on the change: gene - reference gene, the calculated p-value is the probability that at least one of the groups is different from the others. The calculations have been done on 5 potential house-keeping genes, or on the mean of more than one, only the most stable ones have been kept with the criteria of the least residual error.

### Example 2:

An example of the composition of an infant formula for use according to the present invention is given below. This composition is given by way of illustration only. The protein source is a conventional mix of whey protein and casein.

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic (100% GOS) (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *L. reuteri* | 2x10⁷ cfu/g of powder | |
| DSM 17938 | | |

## Claims

1. *Lactobacillus reuteri* DSM 17938 for promoting the healthy development and/or repair of the enteric nervous system in a young mammal.

2. *Lactobacillus reuteri* DSM 17938 according to claim 1, wherein the young mammal is an non-human animal or a pet.

3. *Lactobacillus reuteri* DSM 17938 according to claim 1, wherein the young mammal is a human foetus, pre-term or term born infant, toddler or child.

4. *Lactobacillus reuteri* DSM 17938 according to any one of claims 1-3, wherein the young mammal has experienced or is experiencing IUGR, and/or had, or is predicted to have, a low, very low or extremely low birth weight, and/or is suffering or suffered from enteric nervous system growth retardation either *in utero* or, during or after birth.

5. *Lactobacillus reuteri* DSM 17938 according to claim 4, wherein the enteric nervous system growth retardation is due to hypoxemia-ischemia at birth.

6. *Lactobacillus reuteri* DSM 17938 according to any of the preceding claims, wherein administration to the foetus is via the expectant mother.

7. *Lactobacillus reuteri* DSM 17938 according to claim 6, wherein administration to the expectant mother begins when she is 1, 2, 3, 4, 5, 6, 7, 8 or 9 months pregnant, or equivalent time for administration to an expectant animal.

8. *Lactobacillus reuteri* DSM 17938 according to any of claims 1-5, wherein administration to the young mammal is direct or indirect, via the lactating mother.

9. *Lactobacillus reuteri* DSM 17938 according to any of the preceding claims, wherein administration period to the foetus or infant has a duration of at least one week, preferably two weeks, more preferably at least one month.

10. *Lactobacillus reuteri* DSM 17938 according to any of the preceding claims, wherein administration period to the toddler or young child has a duration of at least 4 weeks, preferably 2-12 months, and more preferably for a period of at least 18 months.

11. *Lactobacillus reuteri* DSM 17938 according to any of the preceding claims, wherein administration period for a young child is up until the child is 4 years old, preferably, 6 years old, and more preferably, 10 years old.

12. *Lactobacillus reuteri* DSM 17938 according to any of the preceding claims, wherein the *Lactobacillus reuteri* DSM 17938 is administered directly to the infant or toddler in its pure form, or diluted in water or breast milk, in a food supplement, or in a milk fortifier, or any enteral feeding including milk support during trophic feeding, in an infant formula, or in a milk based drink.

13. *Lactobacillus reuteri* DSM 17938 according to claim 12, wherein the infant formula is a formula for premature infants, a starter formula or a follow-on formula or a growing-up milk.

14. *Lactobacillus reuteri* DSM 17938 according to any of the preceding claims, wherein administration to the expectant or lactating mother or child is orally, preferably in foods, drinks, dietary supplements or pharmaceutical compositions.

15. *Lactobacillus reuteri* DSM 17938 according to any of claims 1-3 and 8-12, wherein said *Lactobacillus reuteri* DSM 17938 is administered to infant, toddler or child as a daily dose of from 1x10³ to 1x10¹², preferably, 1x10⁷ to 1x10¹¹ cfu (cfu = colony forming unit).

16. *Lactobacillus reuteri* DSM 17938 according to any of the preceding claims, wherein said *Lactobacillus reuteri* DSM 17938 is administered to the expectant or lactating mother, or baby, or toddler or child as a composition comprising between 1 x10³ and 1x10¹² cfu/g of dry composition.

17. *Lactobacillus reuteri* DSM 17938 according to claim 15, wherein said composition comprises further ingredients or prebiotics, preferably selected from inulin, fructooligosaccharide (FOS), short-chain fructooligosaccharide (short chain IOS), galacto-oligosaccharide (GOS) and cow milk oligosaccharides (CMOS).

18. *Lactobacillus reuteri* DSM 17938 according to claim 15, 16 or 17, wherein the composition also comprises one or more additional probiotics.

19. *Lactobacillus reuteri* DSM 17938 according to claim 18, wherein the one or more additional probiotic(s) is/are preferably selected from *Bifidobacterium longum* BB536 (ATCC BAA-999); *Lactobacillus rhamnosus* (CGMCC 1.3724), *Bifidobacterium lactis* (NCC2818) or mixtures thereof.

20. *Lactobacillus reuteri* DSM 17938 according to any of the preceding claims, wherein the said *Lactobacillus reuteri* DSM 17938 has been inactivated such as to render it non-replicating.

21. *Lactobacillus reuteri* DSM 17938 according to any of claims 1 to 18, wherein the healthy development and/or repair of the enteric nervous system is correlated with an increased neuronal stimulatory capacity in the jejunum and/or colon compared to that of non-treated subjects.

22. *Lactobacillus reuteri* DSM 17938 according to any of claims 1 to 19, wherein the healthy development and/or repair of the enteric nervous system is manifested as increased expression of Sox10 compared to that of non-treated subjects.

23. *Lactobacillus reuteri* DSM 17938 according to any of claims 1 to 20, wherein the healthy development and/or repair of the enteric nervous system is manifested as a normal gene expression level of GAP43, Ngfrap1, Ntf4 and S100b.

24. A composition comprising *Lactobacillus reuteri* DSM 17938 for promoting the healthy development and/or repair of the enteric nervous system in a young mammal.

25. The composition according to claim 24, wherein the composition is a food supplement, milk fortifier, a starter infant formula, a follow-on infant formula or a growing-up milk.
